(19) Europäisches Patentamt
European Patent Office
Office européen des brevets

(11) Publication number: **0 086 756 A2**

# (12) EUROPEAN PATENT APPLICATION

(21) Application number: 83830029.1

(22) Date of filing: 09.02.83

(51) Int. Cl.³: **A 61 K 7/06**, C 07 C 153/09

(30) Priority: 12.02.82 IT 1965182

(43) Date of publication of application: 24.08.83 Bulletin 83/34

(84) Designated Contracting States: BE CH DE FR GB LI LU NL SE

(71) Applicant: Farmatis S.r.l., Corso Europa n. 5, I-20100 Milano 1 (IT)

(72) Inventor: Scalesciani, Juan Boris Aldo, Calle J.B. Alberdi, Buenos Aires (AR)

(74) Representative: Gervasi, Gemma et al, Studio Brevetti e Marchi NOTARBARTOLO & GERVASI 33, Viale Bianca Maria, I-20122 Milano (IT)

## (54) Dermatological compositions based on mercaptopropionylamidoacetic thiol esters.

(57) Dermatological compositions having curative antiseborrhoeic and antidandruff action, containing as active principle compounds of the formula

$$R_2CO\text{-}S\text{-}(\underset{\substack{|\\ R_1}}{CH})_n\text{-}\underset{\substack{\|\\ O}}{C}\text{-}NH\text{-}CH_2\text{-}COOH \qquad (I)$$

wherein $R_2$ = alkyl radical with 8 20 carbon atoms; $R_1$ = H or $CH_3$; n = 1 or 2 and salts thereof.

EP 0 086 756 A2

## Dermatological compositions based on mercaptopropionylamidoacetic thiol-esters

This invention deals with new dermatological compositions which have an antiseborrheic and antidandruff curative effect and contain mercaptopropionylamidoacetic acid derivatives as active principle.

In our application for Italian Patent No. 19650 A/82, submitted on the same date, we describe a new class of compounds having the following general formula:

$$R_2CO-S-(\underset{\displaystyle R_1}{\underset{|}{CH}})_n-\underset{\displaystyle O}{\underset{\|}{C}}-NH-CH_2-COOH \qquad (I)$$

in which $R_2$ = alkyl radical with 8-20 atoms of carbon; $R_1$ = H or $CH_3$; n = 1 or 2 or their salts.

The alpha-mercaptopropionylamidoacetic acid derivatives, described and included in formual I are more precisely represented by the following general formula:

$$CH_3 - \underset{\displaystyle S-CO-R_2}{\underset{|}{CH}} - \overset{\displaystyle O}{\overset{\|}{C}} - NH - CH_2 - COOH$$

while the beta-mercaptopropionylamidoacetic acid derivatives, included in formula I, are represented by the following general formula:

$$\underset{\displaystyle S-CO-R_2}{\underset{|}{CH_2}} -, CH_2 - \overset{\displaystyle O}{\overset{\|}{C}} - NH - CH_2 - COOH$$

in which $R_2$ is as has been previously defined.

In the same application for patent that was submitted, we also describe an industrial process for the preparation of new compounds (I), which essentially consists of reacting glycine with a halogenide of alpha-bromo or beta-bromo-propionic acid. The bromo-propionamidoacetic acid thus obtained is then made to react with thioacids having the general formula:

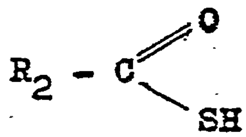

where $R_2$ = alkyl radical, linear or with 8-20 carbon-atom branches. The obtained acids can be saltified with alkaline alcoholates or organic bases. The compounds, (I), are described as mucolytics, hepatoprotectors and antihypertensives suitable for human therapy.

We have even made an unexpected discovery, which constitutes the object of the Patent Application. We have found that the very same compounds with formula (I):

$$R_2-CO-S-(\underset{R_1}{\underset{|}{CH}})_n-\underset{O}{\underset{\|}{C}}-NH-CH_2-COOH$$

where $R_1$, $R_2$ and n are as previously defined, act on the skin and manifests an intensive antiseborrheic action which favors the detersion and hygeine of oily skin which has pimples, blackheads and has a predisposition for developing acne. These compounds also have an antidandruff action which favors the regeneration of weakened and fragile hair.

The antiseborrheic action of the formula I compounds was first observed by administrating to rats which had a hyperseborrheic condition. The compounds were administered orally to rats whose hyperseborrheic condition was due to being fed a biotine-poor (egg-yolk rich) diet. Doses between 0.1 and 0.9 g/kg/die of the compounds of this invention were administered. In particular, formula I compounds containing $R_2$ = laurylic, myristylic or cetylic radical were tested, the compounds being administered by mixing with the food.

In all cases, the results obtained were comparable. Fifteen days following treatment, the seborrheic lesions were reduced in intensity and extension. The fur, moreover, was less greasy and healthier, not sticky, opaque or bristly, in comparison with the control group, and the skin was clearer and not wrinkled. Autoptic examination of the livers of the treated animals proved the livers normal, while those of the control group showed a

hypertrophic condition together with degenerative alterations, which is typical in cases of biotine lack. The histological examinations of the skin of the treated animals showed a decided decrease in the volume of the sebaceous glands, while those of the animals in the control group were enlarged with many of them being ruptured.

The cutaneous lipids determination showed an average of 20% in the treated animals as compared to the control group.

Tests on the formula I compounds were subsequently carried out to determine their capability of reducing sebaceous hypersecretion at the cutaneous level. After making preliminary tests to determine local tolerability, local acute, sùbchronic and systemic toxicity, topical applications were made in the form of an appropriate formulation for the skin.

The abdomens of white rabbits were depilated and local applications were made to determine the degree of skin irritation. The average weight of the rabbits was 1.500 kg and depilated test area measured 12 centimeters in length and 7 centimeters in width.

Skin abrasions were produced at the anterior end of the test area, measuring about 2 $cm^2$, using sandpaper and taking care not to draw blood. About 0.5 g of each of the composition samples, 1 to 4, described in the following section, was uniformly applied to the abrased portion of skin at the anterior end of the test area and to the posterior end of the test area. At the end of 24 hours, the compositions were removed and the test areas immediately inspected for signs of irritation produced by the compositions. In all cases, both the abraded and non-abraded portions of the test areas appreared completely normal. The normal appearance of the test areas remained unaltered at the next inspection, 24 hours later, and the third inspection, 48 hours later.

For carrying out the acute cutaneous toxicity tests, the abdomens of white rabbits were prepared by first depilating a 15 by 5 cm area. Next, abrasions were made on each side of the centerline of the of the test area, 5 abrasion spots in a line on each side of the centerline. The abrasions were made deep enough to involve the corneo strata but not deep enough to cause bleeding.

About 5 grams of each of the example compositions, 1 to 4, described in the following section, was uniformly applied to about a 105 $cm^2$ area on the depilated zone of each animal.

The test areas on the rabbits were then covered with a layer of gauze and held in place by a bandage. At the end of 24 hours, the bandages and gauze were removed and the test areas were inspected. No cutaneous lesions were noted in any of the animals tested.

Subchronic cutaneous toxicity was determined by first preparing a 12 by 7 cm test area on the abdomens of white rabbits and then applying 0.5 grams of the composition examples, 1 to 4, described in the following section, onto the depilated test areas. The applications were made daily for a period of 25 days and the test areas were not covered. At the end of the treatment period, the test areas were inspected for signs of local irritation. None were observed

The systemic affects of the subchronic treatment were determined by taking rectal temperature readings of the animals undergoing this treatment, every seven days. The observed temperature readings remained within ± 1.5°C of the initial temperature readings. The rectal temperature readings of the animals of the control group, which were kenneled under the same conditions as the test animals, remained within the same limits. At the end of the treatment period, the urine of the rabbits treated was individually collected and albumin, bilary pigments and occulated blood tests were carried out, as well as glucose and chetonic body tests. All these tests showed negative. Weights taken during the test period showed that there were no appreciable differences between the weights of the test animals and those of the control group.

Finally, the capacity of the new products to reduce sebaceous hypersecretion at the cutaneous level was tested by inducing hyperseborrheic conditions in white rabbits by feeding them a biotine-free diet.

A test area of 12 x 7 cm was depilated on the abdomen of the rabbits. About 0.5 grams of each of the compositions examples, 1 to 4, described in the next section, was applied to one-half

of the test area of each rabbit while the excipients alone were applied, as a control, on the other half of the test area. The applications were repeated daily for a total period of 25 days. At the end of the test period, the test areas were inspected. The fur on the half of the test area where just the excipients of the compositions 1 to 4, described in the following section, were applied, was much more opaque, bristly and sticky, with the skin more flacid and wrinkled, than was the case with the other half of the test area where the compositions applied contained the active principle described by this invention.

Histological examination of the two skin-area halves, the half treated with the new product and the half treated with just the excipients of the product, revealed a decided contrast between the two. The connective tissue of the areas treated with the new products were well conserved, with good fascicular interweaving, no pathological infiltration and no alterations of any kind of the muscular tissue.

The formulations given below are a few examples of those products which have been amply tested on humans and which, for the soaps, jellies and creams, have proved to have a decided antiseborrheic action favoring the detersion and hygiene of oily skin, thereby inhibiting pimples, blackheads and acne. The shampoos and lotions, on the other hand, which contain the new compounds, have given consistent results in fighting dandruff and provide, as well, a further action which favors the restructuring of weak and fragile hair.

The composition examples given below are purely by was of illustration and, obviously, there are many other compositions possible, using the claimed active products, without exceeding the limits of this invention.

All the compositions have a final pH of 5.5 ± 0.3 and it is to be understood that they all contain a perfume or hypoallergenic aromatic compound, as well as a preservative such as alkyl-p,hydroxylbenzoate, benzylic alcohol and the like. In the case of antiseptic lotions and creams, preservatives are not required, since these products already contain antibacterial agents.

1. <u>CREAM SOAP</u>

| | |
|---|---|
| Alpha-thiopropionylamidoacetic acid esterized with laurylic alcohol | 0.8 g |
| Lecithin | 0.7 g |
| Dodecyl-ethylene-glycol monolaurate | 5.0 g |
| Hamamelis water | 18.0 g |
| Chamomile water | 18.0 g |

Distilled water, q.s., to 100 g.

This soap is suited for the detersion of oily skin having pimples and blackheads, as well as for promoting skin hygiene and treating oily skin having acne problems. Soaps with equivalent action can be prepared by using myristylic or cetylic (palmytic) esters or the corresponding esters of beta-thiopropionylamidoacetic acid.

2. JELLY

| | |
|---|---|
| Alpha-thiopropionylamidoacetic acid esterized with laurylic alcohol | 5.0 g |
| Carboxypolymethylene | 3.0 g |
| Triethanolamine | 3.5 g |
| Isopropanol | 40.0 g |
| Polyoxyethylene(20)-sorbitan-monolaurate | 6.0 g |

Distilled water, q.s., to 100 g.

Myristylic or cetylic (palmytic) thio-esters can also be used with the same good results, as well as can the laurylic, myristylic or cetylic (palmytic) thio-esters of beta-thiopropionylamidoacetic acid.

3. HYDROPHILE UNGUENT

| | |
|---|---|
| Alpha-thiopropionylamidoacetic acid esterized with laurylic alcohol | 2.5 g |
| Polyglycol 4,000 | 11.2 g |
| Stearylic alcohol | 20.8 g |
| Glycerine | 17.1 g |
| Sodium laurylsulphate | 0.6 g |

Distilled water, q.s., to 100 g.

Myristylic or cetylic (palmytic) thio-esters can also be used with the same good results, as well as can the corresponding thio-esters of beta-thiopropionylamidoacetic acid.

Unguents of this type have proved highly effective in the treatment of dry skin with acne tendency.

4. ANTISEPTIC CREAM (emulsified lotion)

| | |
|---|---|
| Alpha-thiopropionylamidoacetic acid esterized with laurylic alcohol | 3.0 g |
| Cetyltrimethylammonium bromide | 0.2 g |
| White wax | 0.1 g |
| Cetylic (palmytic) alcohol | 1.5 g |
| Glycerine | 5.0 g |
| Sodium laurylsulfate | 0.5 g |
| Distilled water, q.s., to 100 g. | |

Myristylic or cetylic (palmytic) thio-esters can also be used with the same good results, as well as can the corresponding esters of beta-thiopropionylamidoacetic acid.

These creams are particularly effective for disinfecting oily skin having pimples and blackheads, as well as for skin hygeine and for the treatment of oily skin having acne tendency.

5. ANTISEPTIC LOTION

| | |
|---|---|
| Alpha-thiopropionylamidoacetic acid esterized with laurylic alcohol | 6.0 g |
| Cetyltrimethylammonium bromide | 0.1 g |
| Polyoxyethylene(20)-sorbitan-monolaurate | 10.0 g |
| Ethyl alcohol, 96%, and water, q.s., to 100g for an alcoholic content of | 12.5° |

Myristylic and cetylic thio-esters can also be used with the same good results, as well as can the corresponding esters of beta-thiopropionylamidoacetic acid.

These compositions are particularly effective for disinfecting oily skin having pimples and blackheads, as well as for skin hygeine and for the treatment of oily skin having acne tendency.

6. CREAM SHAMPOO (for dry and fragile hair)

| | |
|---|---|
| Alpha-thiopropionylamidoacetic acid esterized with laurylic alcohol | 10.0 g |
| Magnesium stearate | 1.0 g |
| Sodium laurylsulfate | 30.0 g |
| Polyvinylic alcohol, 10% | 20.5 g |
| Methylcellulose | 9.0 g |
| Glycerylmonolaurate | 2.0 g |
| Lanolin | 0.2 g |
| Distilled water, q.s., to 100 g. | |

These compositions can be equally effective if myristylic or

cetylic (palmytic) esters are used,or if the esters of beta-thiopropionylamidoacetic acid are used.

7. <u>LIQUID SHAMPOO</u> (for oily hair)

| | |
|---|---|
| Alpha-thiopropionylamidoacetic acid esterized with laurylic alcohol | 10.0 g |
| Triethanolamine laurylsulfate | 35.0 g |
| Sodium alginate | 2.5 g |
| Distilled water, q.s.,to 100 g. | |

Equally effective liquid shampoos can also be prepared using myristylic or cetylic (palmytic) esters or the corresponding esters of beta-thiopropionylamidoacetic acid.

8. <u>LIQUID SHAMPOO</u> (for oily hair)

| | |
|---|---|
| Alpha-thiopropionylamidoacetic acid esterized with laurylic alcohol | 10.0 g |
| Coconut oil | 14.0 g |
| Olive oil | 3.0 g |
| Castor oil | 3.0 g |
| Potassium hydrate, 85% | 4.7 g |
| Glycerine | 2.0 g |
| Ethyl alcohol, 96° | 6.0 g |
| Sodium hexametaphosphate | 1.0 g |
| Distilled water, q.s.,to 100 g. | |

Equally effective shampoos can be obtained by using myristylic or cetylic (palmytic) esters, as well as by using the corresponding esters of beta-thiopropionylamidoacetic acid.

9. <u>HAIR TONIC</u> LOTION

| | |
|---|---|
| Alpha-thiopropionylamidoacetic acid esterized with laurylic alcohol | 5.0 g |
| Amniotic liquid | 1.0 g |
| Polyoxyethylene(20)-sorbitan-monolaurate | 6.0 g |
| Ethyl alcohol, 96° and water, q.s., to 100 g to obtain alcohol content of | 25° |

Equivalent formulations have been prepared using the corresponding myristylic or cetylic (palmytic) thio-esters, as well as the laurylic, myristylic or cetylic (palmytic) thio-esters of beta-thiopropionylamidoacetic acid.

0086756

## Claims

1. Dermotological compositions having antiseborrheic and antidandruff action, characterized by the fact that the compound contained as active principle conforms to the following general formula:

$$R_2\text{-CO-S-}\underset{R_1}{\underset{|}{(CH)}}_n\text{-}\underset{O}{\underset{\|}{C}}\text{-NH-}CH_2\text{-COOH} \qquad (I)$$

where $R_2$ = alkyl radical with 8-20 carbon atoms, $R_1$ = H or $CH_3$; n = 1 or 2 or one of its salts in combination with appropriate excipients and dilutents.

2. A curative method for cutaneous infections due to hyperseborrheic conditions, characterized by the fact that the dermatological compositions, which are applied to the skin, contain as active principle a compound which conforms to the following general formula:

$$R_2\text{-CO-S-}\underset{R_1}{\underset{|}{(CH)}}_n\text{-}\underset{O}{\underset{\|}{C}}\text{-NH-}CH_2\text{-COOH}$$

where $R_2$ = alkyl radical with 8-20 carbon atoms; $R_1$ = H or $CH_3$; n = 1 or 2 or one of its salts in combination with appropriate excipients and dilutents.

3. A method for curing dandruff and regenerating the hair, characterized by the fact that the scalp is treated with compositions containing, as active principle, a compound which conforms to the following general equation:

$$R_2\text{-CO-S-}\underset{R_1}{\underset{|}{(CH)}}_n\text{-}\underset{O}{\underset{\|}{C}}\text{-NH-}CH_2\text{-COOH}$$

where $R_2$ = alkyl radical with 8-20 carbon atoms; $R_1$ = H or $CH_3$; n = 1 or 2 or one of its salts in combination with appropriate exipients and dilutents.